(19) Europäisches Patentamt
European Patent Office
Office européen des brevets

(11) **EP 3 748 345 A1**

(12) **EUROPEAN PATENT APPLICATION**
published in accordance with Art. 153(4) EPC

(43) Date of publication:
**09.12.2020 Bulletin 2020/50**

(51) Int Cl.:
**G01N 23/2055** (2018.01)     **G01L 1/25** (2006.01)

(21) Application number: **18902514.1**

(22) Date of filing: **21.12.2018**

(86) International application number:
**PCT/JP2018/047320**

(87) International publication number:
**WO 2019/146340 (01.08.2019 Gazette 2019/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.01.2018 JP 2018012199**

(71) Applicant: **Kabushiki Kaisha Kobe Seiko Sho
(Kobe Steel, Ltd.)
Kobe-shi, Hyogo 651-8585 (JP)**

(72) Inventors:
• **MATSUDA, Mariko**
  **Takasago-shi, Hyogo 676-8670 (JP)**
• **KABUTOMORI, Tatsuhiko**
  **Takasago-shi, Hyogo 676-8670 (JP)**
• **TAKAMATSU Hiroyuki**
  **Kobe-shi, Hyogo 651-2271 (JP)**

(74) Representative: **Müller-Boré & Partner
Patentanwälte PartG mbB
Friedenheimer Brücke 21
80639 München (DE)**

(54) **RESIDUAL STRESS MEASURING METHOD**

(57)    The present invention is a method for measuring a residual stress in a cast and forged steel product, the method using X-rays, including: irradiating a cast and forged steel product with X-rays; two-dimensionally detecting intensities of diffracted X-rays originating from the X-rays; and calculating a residual stress based on a diffraction ring formed by an intensity distribution of the diffracted X-rays detected in the detecting, wherein, when the residual stress is measured for each of a plurality of measurement positions of the cast and forged steel product, the residual stress for each of the measurement positions is calculated in the calculating based on the diffraction ring for each of the measurement positions and an X-ray elastic constant which varies for each of the measurement positions.

FIG. 1

**Description**

[TECHNICAL FIELD]

The present invention relates to a method for measuring a residual stress.

[BACKGROUND ART]

Recently, a residual stress measurement technique using X-rays has been widely applied. In this technique, a lattice distortion occurring inside a specimen having a crystalline structure is measured using X-rays, and the measurement result is converted into a residual stress.

As a method for measuring a residual stress using X-rays, a cos α method is known. In the cos α method, a specimen is irradiated with X-rays at a specific irradiation angle, intensities of diffracted X-rays generated by reflection of the X-rays by the specimen are two-dimensionally detected, and a residual stress is calculated based on a diffraction ring formed by an intensity distribution of the diffracted X-rays which have been detected. For example, in Patent Document 1, a specific calculation procedure of a residual stress by the cos α method is described.

In an X-ray diffraction system disclosed in Patent Document 1, an X-ray diffraction apparatus is stopped at an arbitrary measurement portion on a rail to perform irradiation with X-rays, diffracted X-rays are detected by an imaging plate, and a residual stress is evaluated based on a diffraction ring formed by the diffracted X-rays (Paragraph [0025]). The X-ray diffraction system in Patent Document 1 can monitor degradation over time of each part of the rail by accumulating measurement data at each measurement point on the rail while moving a vehicle equipped with the X-ray diffraction apparatus and by evaluating an average value of the measurement data at each measurement point (Paragraphs [0057] and [0059]).

Incidentally, a cast and forged steel product may internally have a locally uneven distribution of chemical components depending on production conditions such as a kind of an element contained therein, a concentration of an element contained therein, a cooling rate in solidification of molten steel, and the like. In this case, the cast and forged steel product tends not to be completely uniform in structure and hardness, and a residual stress occurring inside the cast and forged steel product also tends to vary locally. This tendency is particularly significant in a large cast and forged steel product.

In the case of performing an X-ray residual stress measurement using a cast and forged steel product as a specimen, when a non-uniform portion in the cast and forged steel product is selected as a measurement position, a measurement result of the residual stress may include a large error. Hence, there is a demand for a method for measuring a residual stress, which can properly evaluate a residual stress for each measurement position of a cast and forged steel product.

[PRIOR ART DOCUMENTS]

[PATENT DOCUMENTS]

Patent Document 1: Japanese Unexamined Patent Application Publication No. 2005-241308

[SUMMARY OF THE INVENTION]

[PROBLEMS TO BE SOLVED BY THE INVENTION]

The present invention was made in view of the foregoing circumstances, and an object of the present invention is to provide a method for measuring a residual stress which can properly evaluate a residual stress for each measurement position of a cast and forged steel product.

[MEANS FOR SOLVING THE PROBLEMS]

The invention made to solve the aforementioned problems is a method for measuring a residual stress in a cast and forged steel product, the method using X-rays, including: irradiating a cast and forged steel product with X-rays; two-dimensionally detecting intensities of diffracted X-rays originating from the X-rays; and calculating a residual stress based on a diffraction ring formed by an intensity distribution of the diffracted X-rays detected in the detecting, wherein, when the residual stress is measured for each of a plurality of measurement positions of the cast and forged steel product, the residual stress for each of the measurement positions is calculated in the calculating based on the diffraction ring for each of the measurement positions and an X-ray elastic constant which varies for each of the measurement positions.

For calculation of a residual stress in a residual stress measurement using X-rays, an X-ray elastic constant and a diffraction ring formed by an intensity distribution of diffracted X-rays are used. In a general method for measuring a residual stress by use of X-rays, a residual stress is calculated using one standard X-ray elastic constant corresponding to a material of a specimen. However, since the X-ray elastic constant is determined by a chemical component, an internal structure, hardness, and/or the like of the specimen, an X-ray elastic constant of a non-uniform portion in the cast and forged steel product is different from the standard X-ray elastic constant of the cast and forged steel product. Therefore, the general method for measuring a residual stress by use of the X-rays cannot properly evaluate a residual stress occurring in the non-uniform portion in the cast and forged steel product. Meanwhile, in the method for measuring a residual stress of the present invention, the residual stress is calculated using the X-ray elastic constant which varies for each of the measurement positions of the cast and forged steel product, whereby a proper X-ray elastic constant can be selected in accordance with the chemical component, the internal structure, the hardness, and/or the like for each of the measurement positions of the cast and forged steel product. Thus, the method for measuring a residual stress of the present invention can properly evaluate the residual stress for each of the measurement positions of the cast and forged steel product.

The X-ray elastic constant which varies for each of the measurement positions is preferably determined based on at least one of: a half width of the diffracted X-rays originating from the X-rays, a chemical component of the cast and forged steel product, or a hardness of the cast and forged steel product. Thus, the method for measuring a residual stress of the present invention can calculate a residual stress by use of an appropriate X-ray elastic constant for each of the measurement positions of the cast and forged steel product.

The measurement positions are preferably arranged at intervals that fall within five times an irradiation diameter of the X-rays. When the measurement positions are widely separated from one another, the chemical component, the internal structure, the hardness, and/or the like may largely vary between the measurement positions. In the method for measuring a residual stress of the present invention, the measurement positions are arranged at the above intervals, whereby an appropriate X-ray elastic constant can be selected in accordance with a change in the chemical component, the internal structure, the hardness, and/or the like for each of the measurement positions.

[EFFECTS OF THE INVENTION]

The method for measuring a residual stress of the present invention can properly evaluate a residual stress for each measurement position of a cast and forged steel product.

[BRIEF DESCRIPTION OF THE DRAWINGS]

Fig. 1 is a flow chart showing a method for measuring a residual stress of a first embodiment of the present invention.
Fig. 2 is a flow chart showing details of a testing step in Fig. 1.
Fig. 3 is a flow chart showing part of a method for measuring a residual stress of a second embodiment of the present invention.
Fig. 4 is a flow chart showing details of a testing step in Fig. 3.
Fig. 5 is a graph showing a relation between a conventional stress and an X-ray stress measured using a test specimen with much segregation.
Fig. 6 is a graph showing a relation between a conventional stress and an X-ray stress measured using a test specimen with little segregation.
Fig. 7 is a graph showing a relation between a half width of diffracted X-rays and a correction factor used for correction of an X-ray elastic constant.
Fig. 8 is a graph showing a relation between a carbon equivalent of a cast and forged steel product and a correction factor used for correction of an X-ray elastic constant.
Fig. 9 is a graph showing a relation between a Vickers hardness of a cast and forged steel product and a correction factor used for correction of an X-ray elastic constant.

[DESCRIPTION OF EMBODIMENTS]

Hereinafter, embodiments of the method for measuring a residual stress of the present invention will be described in detail with reference to the drawings.

First Embodiment

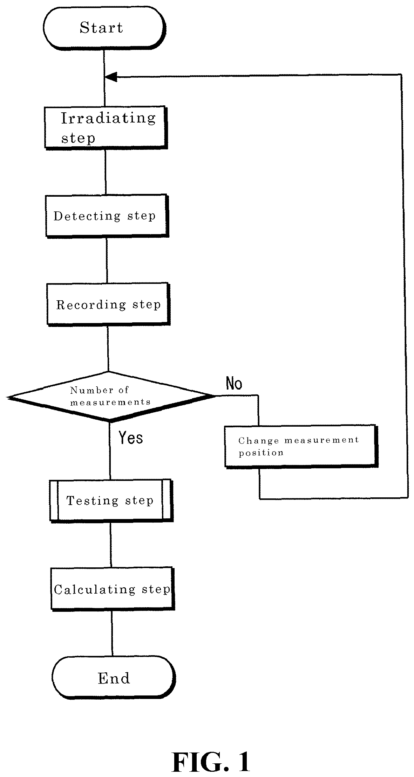

A method for measuring a residual stress shown in Fig. 1 is a method for measuring a residual stress in a cast and forged steel product, the method using X-rays, including: irradiating a cast and forged steel product with X-rays (an irradiating step); two-dimensionally detecting intensities of diffracted X-rays originating from the X-rays (a detecting step); and calculating a residual stress based on a diffraction ring formed by an intensity distribution of the diffracted X-rays detected in the detecting step (a calculating step). In the method for measuring a residual stress, when the residual stress is measured for each of a plurality of measurement positions of the cast and forged steel product, the residual stress for each of the measurement positions is calculated in the calculating step based on the diffraction ring for each of the measurement positions and an X-ray elastic constant which varies for each of the measurement positions. Furthermore, the method for measuring a residual stress further includes, after the detecting step, recording the intensities of the diffracted X-rays detected in the detecting step (a recording step) and, before the calculating step, obtaining correction conditions for correcting the X-ray elastic constant for each of the measurement positions (a testing step).

In the method for measuring a residual stress, an X-ray irradiation apparatus and an X-ray stress measurement apparatus including a two-dimensional detector are used. In the method for measuring a residual stress, an irradiation position at which the cast and forged steel product is irradiated with the X-rays is changed each time the cast and forged steel product is irradiated with the X-rays. Then, in the method for measuring a residual stress, the residual stress for each irradiation position is calculated based on the diffraction ring for each irradiation position and the X-ray elastic constant which varies for each irradiation position. In other words, in the method for measuring a residual stress, the diffraction ring is obtained for each of the plurality of the measurement positions of the cast and forged steel product, and the residual stress is calculated using the X-ray elastic constant which varies for each of the measurement positions.

Irradiating Step

The irradiating step is a step of irradiating the cast and forged steel product with the X-rays from the X-ray irradiation apparatus. In the irradiating step, the cast and forged steel product is irradiated with the X-rays without changing the irradiation position when performing irradiation with the X-rays a first time. Furthermore, in the irradiating step, the irradiation position of the X-rays is changed each time before performing irradiation with the X-rays a second time and thereafter. An amount of change in the irradiation position of the X-rays, which is performed for each irradiation with the X-rays, preferably corresponds to, for example, a distance that falls within five times an irradiation diameter of the X-rays. In other words, the measurement positions are preferably arranged at intervals that fall within five times the irradiation diameter of the X-rays.

It is to be noted that in a case in which there is little variation in the residual stress occurring inside the cast and forged steel product in a wide area, the measurement positions may be arranged at intervals that are greater than five times the irradiation diameter of the X-rays, for example, at intervals that fall within ten times the irradiation diameter of the X-rays. Furthermore, in a case in which there is large variation in the residual stress occurring inside the cast and forged steel product in a narrow area, the measurement positions may be arranged adjacent to one another at intervals that are substantially equal to the irradiation diameter of the X-rays, or may be arranged at intervals that are less than the irradiation diameter of the X-rays such that the measurement positions partly overlap with one another.

Detecting Step

The detecting step is a step of detecting, with a two-dimensional detector, the intensities of the diffracted X-rays originating from the X-rays with which the cast and forged steel product is irradiated. The cast and forged steel product is polycrystalline; therefore, the X-rays with which the cast and forged steel product is irradiated are diffracted by a large number of crystals at angles that satisfy the Bragg's diffraction condition. The X-rays diffracted by the large number of crystals are detected as diffracted X-rays by the two-dimensional detector. The two-dimensional detector detects the intensities of the diffracted X-rays, and an intensity distribution of the diffracted X-rays forms a diffraction ring.

Recording Step

The recording step is a step of recording the intensities of the diffracted X-rays, which have been detected in the detecting step, for each irradiation position of the X-rays, that is, for each of the measurement positions of the cast and forged steel product. In the recording step, when the intensities of the diffracted X-rays, which have been detected in the detecting step, are recorded, X-ray diffraction information relating to the intensities of the diffracted X-rays in the two-dimensional detector is initialized.

Testing Step

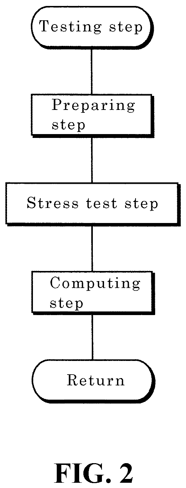

The testing step is a step of obtaining the correction conditions for correcting the X-ray elastic constant for each of the measurement positions of the cast and forged steel product. As shown in Fig. 2, the testing step includes: preparing a test specimen (a preparing step); performing a stress test on the test specimen (a stress test step); and computing a correction factor for correcting the X-ray elastic constant based on the result of the stress test (a computing step).

Preparing Step

In the preparing step, which is a step of preparing the test specimen from the cast and forged steel product, the cast and forged steel product is processed so that the test specimen includes the plurality the measurement positions for which the intensities of the diffracted X-rays have been recorded in the recording step. The preparing step may be a step of processing the cast and forged steel product so that one test specimen includes the plurality of the measurement positions, or a step of processing the cast and forged steel product so that each of a plurality of test specimens includes a corresponding one of the plurality of the measurement positions. It is to be noted that in a case where the stress test can be performed on the cast and forged steel product itself and the cast and forged steel product does not need to be processed, the preparing step may be omitted.

Stress Test Step

The stress test step is a step of performing the stress test on the test specimen. Specifically, in the stress test step, the plurality of the measurement positions of the test specimen is irradiated with the X-rays in a state in which a known stress is applied to the test specimen by a tension tester or the like, the intensities of the diffracted X-rays originating from the X-rays are detected, and the residual stress for each of the measurement positions is calculated from the intensity distribution of the diffracted X-rays. As a method for calculating the residual stress based on the intensity distribution of the diffracted X-rays, a calculation method according to the cos $\alpha$ method is used; however, a method in which a stress is directly calculated from an X-ray distortion may also be used. Furthermore, a standard X-ray elastic constant is used for calculation of the residual stress in the stress test step.

Computing Step

The computing step is a step of computing the correction factor for correcting the X-ray elastic constant based on the result of the stress test. Specifically, in the computing step, a ratio of the residual stress for each of the measurement positions to the known stress in the stress test is computed, and the ratio is obtained as the correction factor of the X-ray elastic constant for each of the measurement positions. It is to be noted that in a case in which a plurality of ratios is computed in the stress test using a plurality of known stresses, in light of increasing accuracy, the correction factor is preferably obtained by averaging the plurality of the ratios for each of the measurement positions.

Calculating Step

The calculating step is a step of calculating, based on the diffraction ring formed by the intensity distribution of the diffracted X-rays detected by the two-dimensional detector, the residual stress for each irradiation position of the X-rays, that is, for each of the measurement positions of the cast and forged steel product. In the calculating step, the residual stress for each of the measurement positions is calculated based on the diffraction ring for each of the measurement positions and the X-ray elastic constant which varies for each of the measurement positions. Specifically, in the calculating step, based on the correction factor of the X-ray elastic constant for each of the measurement positions, wherein the correction factor has been obtained in the computing step, the X-ray elastic constant is corrected for each of the measurement positions, and the residual stress for each of the measurement positions is calculated using the X-ray elastic constant, which has been corrected, and the diffraction ring. As a method for calculating the residual stress based on the diffraction ring, a calculation method according to the cos $\alpha$ method is used; however, a method in which a stress is directly calculated from an X-ray distortion may also be used.

The steps of the method for measuring a residual stress are performed by the following procedure. First, in the method for measuring a residual stress, the irradiating step, the detecting step, and the recording step are performed. In a case in which a number of times of irradiation with the X-rays in total, that is, a number of measurements in total has not reached a prescribed value, the irradiation position of the X-rays, that is, the measurement position is changed, and then the irradiating step, the detecting step, and the recording step are performed again. Meanwhile, in a case in which the number of measurements in total has reached the prescribed value, the testing step and the calculating step are performed.

Advantages

In the method for measuring a residual stress, the residual stress is calculated using the X-ray elastic constant, which varies for each of the measurement positions of the cast and forged steel product irradiated with the X-rays; therefore, even in a case in which a measurement position including a non-uniform portion in the cast and forged steel product is irradiated with the X-rays, an appropriate X-ray elastic constant can be selected for the measurement position of the cast and forged steel product. Thus, the method for measuring a residual stress can properly evaluate the residual stress for each of the measurement positions of the cast and forged steel product.

Second Embodiment

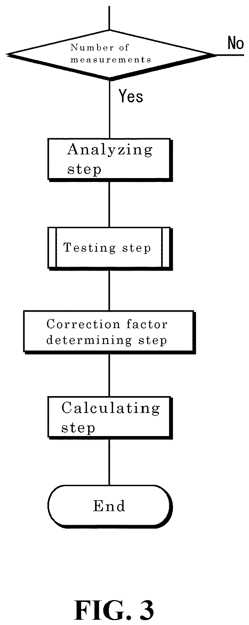

The method for measuring a residual stress of the second embodiment is a method for measuring a residual stress in a cast and forged steel product by use of X-rays, and is different from the method for measuring a residual stress of the first embodiment in that the method for measuring a residual stress of the second embodiment further includes, before the testing step, analyzing at least one property of the cast and forged steel product for each of the measurement positions (an analyzing step), and, after the testing step and before the calculating step, determining a correction factor which varies for each of the measurement positions (a correction factor determining step). The method for measuring a residual stress of the second embodiment is similar to the method for measuring a residual stress of the first embodiment in terms of the irradiating step, the detecting step, and the recording step, and is different from the method for measuring a residual stress of the first embodiment in terms of a process shown in Fig. 3, the process being performed in a case in which a number of measurements in total has reached a prescribed value. The differences from the method for measuring a residual stress of the first embodiment will be described below.

Analyzing Step

The analyzing step is a step of analyzing a parameter indicating the at least one property of the cast and forged steel product for each of the measurement positions. As the parameter indicating the at least one property of the cast and forged steel product, at least one of a half width of the diffracted X-rays, a chemical component of the cast and forged steel product, or a hardness of the cast and forged steel product is used. In the analyzing step, at least one of the half width of the diffracted X-rays, the chemical component of the cast and forged steel product, or the hardness of the cast and forged steel product is analyzed for each irradiation position of the X-rays, that is, for each of the measurement positions.

Testing Step

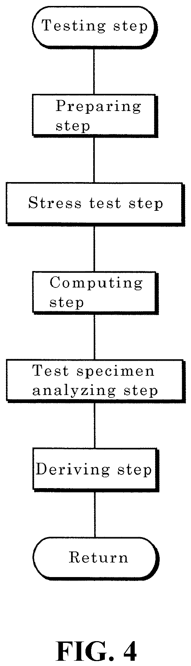

The testing step is a step of obtaining correction conditions for correcting an X-ray elastic constant. As shown in Fig. 4, the testing step includes: preparing a test specimen (a preparing step); performing a stress test on the test specimen (a stress test step); computing a correction factor for correcting the X-ray elastic constant based on the result of the stress test (a computing step); analyzing at least one property of the test specimen (a test specimen analyzing step); and deriving a relational expression between the correction factor computed in the computing step and the at least one property of the test specimen analyzed in the test specimen analyzing step (a deriving step). It is to be noted that the test specimen, which is separate from the cast and forged steel product, is used in the testing step; therefore, the testing step may be performed before other steps such as the analyzing step and the like.

Preparing Step

The preparing step is a step of preparing a test specimen equivalent to the cast and forged steel product. In general, the cast and forged steel product is provided with excess material for testing material properties of the product; therefore, the test specimen is preferably taken from the excess material.

Stress Test Step

The stress test step is a step of performing the stress test on the test specimen. Specifically, in the stress test step, an arbitrary plurality of measurement positions of the test specimen is irradiated with the X-rays in a state in which a known stress is applied to the test specimen by a tension tester or the like, the intensities of the diffracted X-rays originating from the X-rays are detected, and the residual stress for each of the measurement positions is calculated from the intensity distribution of the diffracted X-rays. As a method for calculating the residual stress based on the intensity distribution of the diffracted X-rays, a calculation method of the cos $\alpha$ method is used; however, a method in which a stress is directly calculated from an X-ray distortion may also be used. Furthermore, a standard X-ray elastic constant is used for calculation of the residual stress in the stress test step.

Computing Step

The computing step is a step of computing a correction value of the X-ray elastic constant based on the result of the stress test. Specifically, in the computing step, a ratio of the residual stress for each of the measurement positions to the known stress in the stress test is computed, and the ratio is obtained as the correction value of the X-ray elastic constant for each of the measurement positions. It is to be noted that, in a case in which a plurality of ratios is computed in the stress test using a plurality of known stresses, in light of increasing accuracy, the correction value is preferably obtained by averaging the plurality of ratios for each of the measurement positions.

Test Specimen Analyzing Step

The test specimen analyzing step is a step of analyzing a parameter indicating at least one property of the test specimen. Specifically, in the test specimen analyzing step, at least one of a half width of the diffracted X-rays, a chemical component of the test specimen, or a hardness of the test specimen is analyzed for each of the measurement positions.

Deriving Step

The deriving step is a step of deriving the relational expression between the correction value computed in the computing step and the at least one property of the test specimen analyzed in the test specimen analyzing step. The correction value is connected to the parameter indicating the at least one property of the test specimen via the measurement position. In the deriving step, a data group, in which the correction value is plotted on the vertical axis and the parameter indicating the at least one property is plotted on the horizontal axis, is subjected to a least squares approximation by a quadratic function, whereby an approximate curve is obtained, and an equation expressing the approximate curve is derived as the relational expression.

Correction Factor Determining Step

The correction factor determining step is a step of determining the correction factor for correcting the X-ray elastic constant for each of the measurement positions of the cast and forged steel product. In the correction factor determining step, the correction factor which varies for each of the measurement positions of the cast and forged steel product is determined based on the parameter indicating the at least one property of the cast and forged steel product for each of the measurement positions, wherein the parameter has been analyzed in the analyzing step, and the relational expression derived in the deriving step of the testing step. Specifically, in the correction factor determining step, for each of the measurement positions, the parameter indicating the at least one property of the cast and forged steel product, wherein the parameter has been analyzed in the analyzing step, is assigned to the relational expression derived in the deriving step, whereby the correction factor which varies for each of the measurement positions is determined.

Calculating Step

The calculating step is a step of calculating the residual stress for each irradiation position of the X-rays, that is, for each of the measurement positions of the cast and forged steel product, on the basis of the diffraction ring formed by the intensity distribution of the diffracted X-rays detected by a two-dimensional detector. In the calculating step, the residual stress for each of the measurement positions is calculated based on the diffraction ring for each of the measurement positions and the X-ray elastic constant which varies for each of the measurement positions. Specifically, in the calculating step, the X-ray elastic constant is corrected for each of the measurement positions on the basis of the correction factor which varies for each of the measurement positions, wherein the correction factor has been determined in the correction factor determining step, and the residual stress for each of the measurement positions is calculated using the X-ray elastic constant, which has been corrected, and the diffraction ring.

Advantages

In the method for measuring a residual stress, at least one of the half width of the diffracted X-rays, the chemical component of the cast and forged steel product, or the hardness of the cast and forged steel product is analyzed for each irradiation position of the X-rays in the cast and forged steel product. Then, in the method for measuring a residual stress, the X-ray elastic constant which varies for each irradiation position is computed based on the analysis result, and the residual stress for each irradiation position is calculated based on the X-ray elastic constant. Hence, in the method for measuring a residual stress, even in a case in which a region including a non-uniform portion in the cast and forged steel product is irradiated with the X-rays, the residual stress can be calculated using the proper X-ray elastic constant for each irradiation position of the cast and forged steel product. Thus, the method for measuring a residual stress can properly evaluate the residual stress for each of the measurement positions of the cast and forged steel product.

Furthermore, since the half width of the diffracted X-rays, the chemical component of the cast and forged steel product, and the hardness of the cast and forged steel product are non-destructively measurable parameters, the method for measuring a residual stress can properly evaluate the residual stress for each of the measurement positions without destroying the cast and forged steel product to be measured. Moreover, in the method for measuring a residual stress, when the half width of the diffracted X-rays is selected as the parameter indicating the at least one property of the cast and forged steel product, only the intensities of the diffracted X-rays for each of the measurement positions, wherein the intensities have been recorded in the recording step, need to be analyzed in the analyzing step, whereby the analysis can be simplified.

Other Embodiments

It should be understood that the embodiments disclosed herein are illustrative and non-restrictive in every respect. The scope of the present invention is not limited to the structures of the above embodiments, is defined by the terms of the claims, and is intended to include any modifications within the scope and meaning equivalent to the terms of the claims.

In the first and second embodiments described above, the calculating step is performed after the number of times of irradiation with the X-rays in total, that is, the number of measurements in total reaches the prescribed value; however, the calculating step may be performed for each irradiation with the X-rays. In other words, in the method for measuring a residual stress, in a case in which the number of measurements in total does not reach the prescribed value after the irradiating step, the detecting step, and the calculating step are performed, the measurement position may be changed, and then the irradiating step, the detecting step, and the calculating step may be performed again. In this case, the method for measuring a residual stress does not necessarily need to include the recording step.

Furthermore, in the first embodiment described above, the method for measuring a residual stress includes the testing step; however, the first embodiment does not necessarily need to include the testing step. In other words, the method for measuring a residual stress of the first embodiment needs to include at least the irradiating step, the detecting step, and the calculating step. For example, in a case in which an equivalent cast and forged steel product having material properties substantially equal to those of the cast and forged steel product can be prepared, a relation between a measurement position and a correction factor of an X-ray elastic constant of the equivalent cast and forged steel product can be derived in advance by a procedure identical to that of the testing step. In a case in which the relation between the measurement position and the correction factor is derived in advance using the equivalent cast and forged steel product, the testing step can be omitted from the method for measuring a residual stress of the first embodiment because the X-ray elastic constant used in the calculating step can be corrected for each measurement position on the basis of the relation derived in advance. Furthermore, besides such a case, the testing step may be omitted by selecting, for each of the measurement positions, a proper X-ray elastic constant from a plurality of X-ray elastic constants prepared in advance. It is to be noted that, in light of selecting a proper X-ray elastic constant with high accuracy, the method for measuring a residual stress of the first embodiment preferably includes the testing step.

Furthermore, in the second embodiment described above, the method for measuring a residual stress includes the analyzing step, the testing step, and the correction factor determining step; however, the second embodiment does not necessarily need to include the testing step. In the testing step of the second embodiment, the relational expression between the correction value of the X-ray elastic constant and the at least one property of the test specimen is derived in the deriving step, and the relational expression derived once can be reused; therefore, in a case in which the relational expression has already been derived, the testing step can be omitted from the second embodiment.

In the first embodiment described above, the testing step includes the preparing step of preparing the test specimen from the cast and forged steel product; however, in a case in which the stress test can be performed on the cast and forged steel product itself and the preparing step is omitted, the testing step of the first embodiment may be performed at any timing before the calculating step. For example, the testing step of the first embodiment may be performed before the irradiating step.

In the second embodiment described above, the testing step includes the preparing step of preparing the test specimen equivalent to the cast and forged steel product; however, the testing step of the second embodiment may be performed at any timing before the correction factor determining step. For example, the testing step of the second embodiment may be performed before the irradiating step.

In the second embodiment described above, the analyzing step is performed in the case in which the number of measurements in total has reached the prescribed value; however, the analyzing step of the second embodiment may be performed at any time before the correction factor determining step. For example, the analyzing step of the second embodiment may be performed before the irradiating step or may be performed after the detecting step.

In the second embodiment described above, the test specimen equivalent to the cast and forged steel product is prepared in the preparing step; however, as in the first embodiment, the test specimen may be prepared from the cast and forged steel product by processing the cast and forged steel product in the preparing step so that the test specimen includes the plurality of the measurement positions, for which the intensities of the diffracted X-rays have been recorded. In this case, the parameter indicating the at least one property of the cast and forged steel product has already been analyzed in the analyzing step; therefore, the test specimen analyzing step can be omitted from the second embodiment.

[EXAMPLES]

Hereinafter, the present invention will be described more in detail by way of Examples; however, the present invention is not limited to the Examples.

Tension Test of Test Specimen

First, a test specimen with much segregation and a test specimen with little segregation were cut out from a large cast and forged steel product having a weight of greater than 1 t. As the large cast and forged steel product, chromium-molybdenum-based alloy steel having a bainite structure was used. Furthermore, on the basis of knowledge that a black line is observed in a portion with much segregation of a cast and forged steel product, a portion with much segregation and a portion with little segregation were distinguished from each other by use of photography for macroscopic structure observation of the large cast and forged steel product. Furthermore, as the test specimens, rods each including a plate-shaped portion with a length of 70 mm, a width of 12.5 mm, and a thickness of 3 mm in a middle were cut out, and the plate-shaped portions of the test specimens, which had been cut out, were electrolytically polished to a thickness of approximately 0.1 mm.

A test was carried out in which a tension tester was used and, in a state where a tensile stress was applied in a longitudinal direction of each of two kinds of test specimens (the test specimen with much segregation and the test specimen with little segregation), a conventional stress obtained from a load cell of the tension tester was compared with a residual stress measured using X-rays (hereinafter, referred to as "X-ray stress"). Furthermore, as measurement positions of the X-ray stress, $3 \times 3$, i.e., nine points were set at regular intervals in a 6 mm $\times$ 6 mm region of the plate-shaped portion of each of the test specimens, which had been electrolytically polished.

Chromium K$\alpha$ rays were used as the X-rays, a collimator diameter was set to 1.0 mm, an X-ray irradiation distance was set to 80 mm, an X-ray irradiation angle with respect to the test specimens was set to 35°, and an X-ray irradiation area was set to approximately 6.5 mm$^2$. Furthermore, diffracted X-rays from a (211) plane of iron were detected by a two-dimensional detector. As an X-ray elastic constant used for calculation of a residual stress from an obtained diffraction ring, a standard constant employed for a steel material was used. Specifically, a Young's modulus E and a Poisson's ratio v, which were used for calculation of the X-ray elastic constant, were set to 224 GPa and 0.28, respectively.

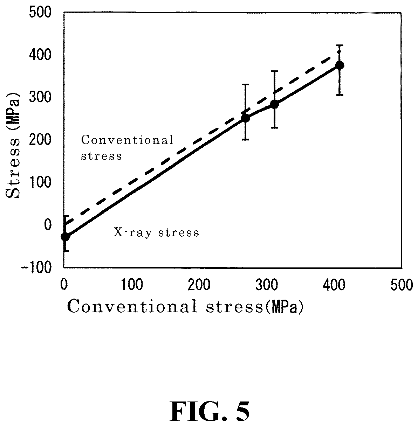

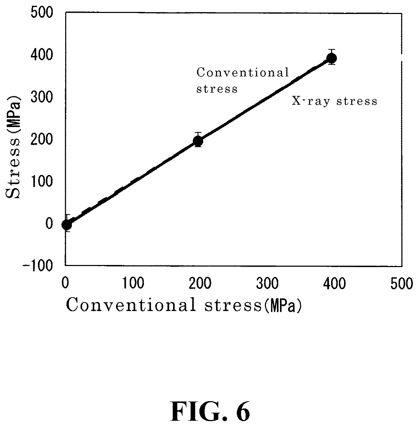

An X-ray stress of the test specimen with much segregation was measured at conventional stresses of 0 MPa, 269 MPa, 312 MPa, and 409 MPa. Furthermore, an X-ray stress of the test specimen with little segregation was measured at conventional stresses of 0 MPa, 197 MPa, and 396 MPa. Fig. 5 is a graph showing a relation between the conventional stress and the X-ray stress measured using the test specimen with much segregation, and Fig. 6 is a graph showing a relation between the conventional stress and the X-ray stress measured using the test specimen with little segregation. It is to be noted that solid lines in the graphs each represent an average value of X-ray stresses at the nine measurement positions, dashed lines in the graphs each represent the conventional stress, and ends of a line extending vertically represent a maximum value and a minimum value of the X-ray stresses at the nine measurement positions.

As shown in Fig. 5, a difference between the maximum value and the minimum value of the X-ray stress in the test specimen with much segregation was confirmed to be very large: the difference was approximately 80 MPa at the conventional stress of 0 MPa and was greater than or equal to 100 MPa at the conventional stresses other than 0 MPa. For further examination, a percentage obtained by dividing the difference between the maximum value and the minimum value of the X-ray stress by the conventional stress was defined as a measurement error of the X-ray stress; the measurement error in the test specimen with much segregation was confirmed to be a very large value, at approximately 49% at the conventional stress of 269 MPa. Furthermore, as shown in Fig. 6, it was confirmed that the difference between the maximum value and the minimum value of the X-ray stress was not small in the test specimen with little segregation, either. A measurement error of the X-ray stress in the test specimen with little segregation was also examined; it was confirmed that the measurement error was not small, being approximately 17% at the conventional stress of 197 MPa.

As described above, it was confirmed that the X-ray stress largely varied depending on the measurement position. In particular, the variation in the X-ray stress in the test specimen with much segregation is extremely large. The variation can be reduced by calculating the X-ray stress by use of the X-ray elastic constant, which varies for each of the measurement positions. Therefore, a method was examined in which the X-ray elastic constant, which varied for each of the measurement positions, was computed, and the X-ray stress was calculated using the X-ray elastic constant.

Analysis of Test Specimen

Properties of the above-described test specimen with much segregation were analyzed for each of the nine measurement positions. As parameters indicating the properties of the test specimen, a half width of the diffracted X-rays, a chemical component of the test specimen, and a hardness of the test specimen were employed. As the half width of the diffracted X-rays, a difference $\Delta B$ between: an average value of half widths of the diffracted X-rays for the measurement positions, wherein the half widths had been obtained in the above-described measurement using the X-rays; and the half width of the diffracted X-rays for each of the measurement positions was employed. As the chemical component of the test specimen, carbon equivalent Ceq computed by an equation (1) below was employed. Furthermore, as the hardness of the test specimen, a Vickers hardness Hv was employed.

$$Ceq = C + \frac{Mn}{6} + \frac{Si}{24} + \frac{Ni}{40} + \frac{Cr}{5} + \frac{Mo}{4} + \frac{V}{4} \quad \cdots (1)$$

In the equation, C denotes a carbon content, Mn denotes a manganese content, Si denotes a silicon content, Ni denotes a nickel content, Cr denotes a chromium content, Mo denotes a molybdenum content, and V denotes a vanadium content, wherein the content of each element is expressed in percent by mass.

Stress Test of Test Specimen

With regard to the above-described test specimen with much segregation, the X-ray stress was measured for the nine measurement positions in a state in which a known stress was applied. Then, a ratio between the known stress and the X-ray stress for each of the measurement positions was computed, and a correction factor $\lambda$ of the X-ray elastic constant for each of the measurement positions was obtained based on the ratio. It is to be noted that the standard Young's modulus E and the standard Poisson's ratio v were used for calculation of the X-ray stress.

Derivation of Relational Expression

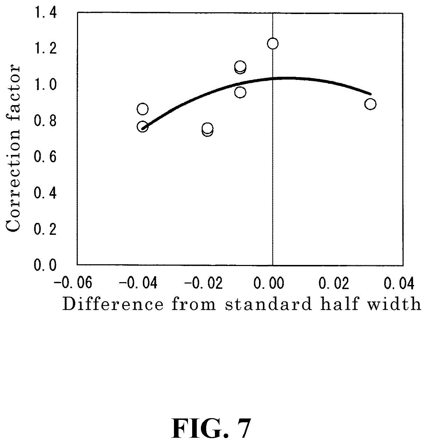

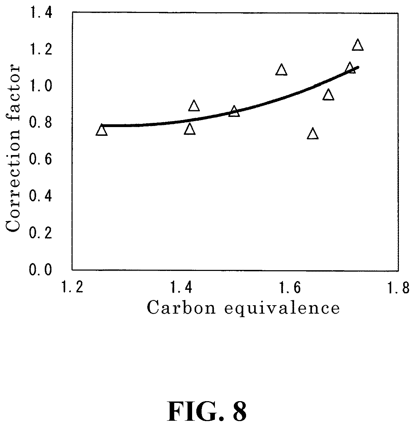

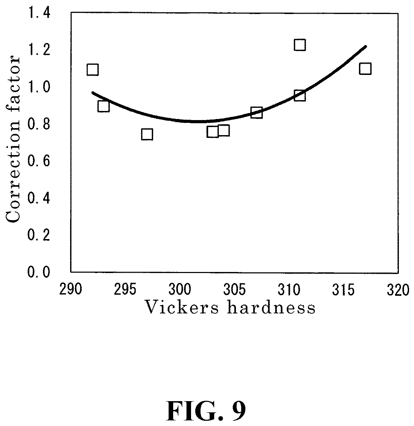

A data group was formed, in which the correction factor $\lambda$ of the X-ray elastic constant obtained for each of the measurement positions was plotted on the vertical axis; and the difference $\Delta B$ between the average value of the half widths of the diffracted X-rays obtained for the measurement positions and the half width of the diffracted X-rays for each of the measurement positions, the carbon equivalent Ceq of the test specimen, or the Vickers hardness Hv of the test specimen was plotted on the horizontal axis. The data group was subjected to a least squares approximation by a quadratic function, whereby an approximate curve serving as a relational expression was derived. Fig. 7 is a graph showing a relation between the correction factor $\lambda$ and the difference $\Delta B$, Fig. 8 is a graph showing a relation between the correction factor $\lambda$ and the carbon equivalent Ceq, and Fig. 9 is a graph showing a relation between the correction factor $\lambda$ and the Vickers hardness Hv. In Fig. 7, the difference $\Delta B$ is expressed by "difference from standard half width". An equation (2) below is a relational expression obtained from Fig. 7, an equation (3) below is a relational expression obtained from Fig. 8, and an equation (4) below is a relational expression obtained from Fig. 9:

$$\lambda = -139.17(\Delta B)^2 + 1.394(\Delta B) + 1.0354 \qquad (2)$$

$$\lambda = 1.6009(Ceq)^2 - 4.0859(Ceq) + 3.3889 \qquad (3)$$

$$\lambda = 0.0017(Hv)^2 - 1.0239(Hv) + 155.17 \qquad (4).$$

Calculation of X-Ray Stress

The correction factor $\lambda$ for each of the measurement positions was determined based on the difference $\Delta B$ for each of the measurement positions and the relational expression represented by the above equation (2), and the X-ray elastic constant for each of the measurement positions was computed based on the correction factor $\lambda$. Then, the X-ray stress for each of the measurement positions was calculated based on the diffraction ring formed by the intensity distribution of the diffracted X-rays for each of the measurement positions, wherein the intensity distribution had been obtained by the measurement using the X-rays, and the X-ray elastic constant obtained for each of the measurement positions. It was confirmed that the X-ray stress calculated as above showed a smaller measurement error and less variation than those of an X-ray stress calculated using a standard X-ray elastic constant.

The correction factor $\lambda$ for each of the measurement positions was determined based on the carbon equivalent Ceq for each of the measurement positions and the relational expression represented by the above equation (3), and the X-ray elastic constant for each of the measurement positions was computed based on the correction factor $\lambda$. Then, the X-ray stress for each of the measurement positions was calculated based on the diffraction ring formed by the intensity distribution of the diffracted X-rays for each of the measurement positions, wherein the intensity distribution had been obtained by the measurement using the X-rays, and the X-ray elastic constant obtained for each of the measurement positions. It was confirmed that the X-ray stress calculated as above showed a smaller measurement error and less variation than those of the X-ray stress calculated using the standard X-ray elastic constant.

The correction factor $\lambda$ for each of the measurement positions was determined based on the Vickers hardness Hv for each of the measurement positions and the relational expression represented by the above equation (4), and the X-ray elastic constant for each of the measurement positions was computed based on the correction factor $\lambda$. Then, the X-ray stress for each of the measurement positions was calculated based on the diffraction ring formed by the intensity distribution of the diffracted X-rays for each of the measurement positions, wherein the intensity distribution had been obtained by the measurement using the X-rays, and the X-ray elastic constant obtained for each of the measurement positions. It was confirmed that the X-ray stress calculated as above showed a smaller measurement error and less variation than those of the X-ray stress calculated using the standard X-ray elastic constant.

[INDUSTRIAL APPLICABILITY]

The method for measuring a residual stress of the present invention can properly evaluate a residual stress at each measurement position of a cast and forged steel product.

**Claims**

1.  A method for measuring a residual stress in a cast and forged steel product, the method using X-rays, comprising:

    irradiating a cast and forged steel product with X-rays;
    two-dimensionally detecting intensities of diffracted X-rays originating from the X-rays; and
    calculating a residual stress based on a diffraction ring formed by an intensity distribution of the diffracted X-rays detected in the detecting, wherein
    when the residual stress is measured for each of a plurality of measurement positions of the cast and forged steel product, the residual stress at each of the measurement positions is calculated in the calculating based on the diffraction ring at each of the measurement positions and an X-ray elastic constant which varies for each of the measurement positions.

2.  The method for measuring a residual stress according to claim 1, wherein the X-ray elastic constant which varies for each of the measurement positions is determined based on at least one of: a half width of the diffracted X-rays originating from the X-rays, a chemical component of the cast and forged steel product, or a hardness of the cast and forged steel product.

3.  The method for measuring a residual stress according to claim 1 or 2, wherein the measurement positions are arranged at intervals that fall within five times an irradiation diameter of the X-rays.

Start

Irradiating step

Detecting step

Recording step

Number of measurements

No

Yes

Change measurement position

Testing step

Calculating step

End

**FIG. 1**

Testing step

Preparing
step

Stress test step

Computing
step

Return

# FIG. 2

**FIG. 3**

Testing step

Preparing
step

Stress test step

Computing
step

Test specimen
analyzing step

Deriving step

Return

# FIG. 4

**FIG. 5**

**FIG. 6**

**FIG. 7**

**FIG. 8**

**FIG. 9**

# INTERNATIONAL SEARCH REPORT

| International application No. |
|---|
| PCT/JP2018/047320 |

| A. CLASSIFICATION OF SUBJECT MATTER |
|---|
| Int.Cl. G01N23/2055(2018.01)i, G01L1/25(2006.01)i |

According to International Patent Classification (IPC) or to both national classification and IPC

| B. FIELDS SEARCHED |
|---|
| Minimum documentation searched (classification system followed by classification symbols) |
| Int.Cl. G01N23/00-23/2276, G01L1/25 |

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII)

| C. DOCUMENTS CONSIDERED TO BE RELEVANT |
|---|

| Category* | Citation of document, with indication, where appropriate, of the relevant passages | Relevant to claim No. |
|---|---|---|
| A | JP 2005-241308 A (RAILWAY TECHNICAL RESEARCH INSTITUTE) 08 September 2005 (Family: none) | 1-3 |
| A | 兼松義一、他，可搬型 X 線回折装置によるレール軸力評価に向けた基礎検討，鉄道総研報告，June 2016, vol. 30, no. 6, pp. 17-22, (KANEMATSU, Yoshikazu et al., "Fundamental Study of Rail Axial Force Evaluation by Portable X-ray Diffraction Device", RTRI REPORT) | 1-3 |
| A | JP 8-201193 A (TOYOTA MOTOR CORP.) 09 August 1996 (Family: none) | 1-3 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 15 March 2019 (15.03.2019) | 26 March 2019 (26.03.2019) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2018/047320 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | 坂井田喜久、他，クロムモリブデン鋼の浸炭硬化層の機械的および X線的弾性定数に及ぼす炭素濃度の影響，材料, May 2013, vol. 62, no. 5, pp. 335-341, (SAKAIDA, Yoshihisa et al., "Influence of Carbon Content on Mechanical and X-Ray Elastic Constants of Carburized Case Layer in Chrome Molybdenum Steel", Journal of the Society of Materials Science, Japan) | 1-3 |
| A | US 5148458 A (RUUD, Clayton) 15 September 1992 (Family: none) | 1-3 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005241308 A